# EUROPEAN PATENT SPECIFICATION

(11) **EP 1 084 708 B1**
(45) Date of publication and mention of the grant of the patent: **19.10.2011**
(21) Application number: 99924651.5
(22) Date of filing: 08.06.1999
(51) Int. Cl.: A61K 36/232, A61K 36/17, A61K 36/45, A61K 36/14, A61K 36/254, A61K 36/67, A61K 36/752, A61K 36/185, A61K 36/282, A61K 36/11, A61K 36/47, A61K 36/54, A61K 36/15, A61K 36/9068

(54) **METHOD OF PROCESSING MEDICATED BATH LIQUOR OF NATURAL PLANT INTO MEDICINAL POWDER**
VERFAHREN ZUR UMWANDLUNG EINER WIRKSTOFFLÖSUNG AUF DER BASIS VON NATURPFLANZEN IN EIN PULVERFÖRMIGES MEDIKAMENT
PROCEDE DE TRANSFORMATION D'UNE SOLUTION MEDICINALE A BASE DE PLANTES NATURELLES EN MEDICAMENT SOUS FORME DE POUDRE

(30) Priority: 11.06.1998 CN 98102252
(43) Date of publication of application: 21.03.2001
(73) Proprietor: Tibet Cheezheng Tibetan Medicine Co. Ltd., Bayi Town, Linzhi Tibet (CN)
(72) Inventor: LEI, Jufang, Lhasa, Tibet 850000 (CN)
(74) Representative: Luderschmidt, Schüler & Partner
(86) International application number: PCT/CN1999/000077
(87) International publication number: WO 1999/064026

(56) References cited:
- CN-A- 1 038 027
- CN-A- 1 038 584
- CN-A- 1 046 284
- DATABASE WPI Section Ch, Week 199822 Derwent Publications Ltd., London, GB; Class B04, AN 1998-249318 XP002278740 & RU 2 092 173 C (DON MED INST), 10 October 1997 (1997-10-10)
- DATABASE WPI Section Ch, Week 199417 Derwent Publications Ltd., London, GB; Class B04, AN 1994-143011 XP002278741 & SU 1 796 188 A (KIEV MED INST), 23 February 1993 (1993-02-23)
- DATABASE WPI Section Ch, Week 199247 Derwent Publications Ltd., London, GB; Class D13, AN 1992-388181 XP002278742 & KR 9 107 636 B (KOREA FOOD RES INST), 28 September 1991 (1991-09-28)
- DATABASE WPI Section Ch, Week 199818 Derwent Publications Ltd., London, GB; Class B04, AN 1998-205419 XP002278743 & RU 2 089 210 C (E MEDICINE CENTRE PRODN ASSOC), 10 September 1997 (1997-09-10)
- DATABASE EPODOC [Online] EUROPEAN PATENT OFFICE, THE HAGUE, NL; JINGYE DING; YOUJIANG LI: "Process for extracting effective component of xizang oriental wormwood, equipment and its tablet preparation method" XP002278739 & CN 1 070 341 A 31 March 1993 (1993-03-31)

## Description

### Field of the Invention

The present invention relates to a method of processing a powdered drug, especially to a method of processing a powdered drug from a medicated bath liquor (MBL) of natural plants.

### Background of the Technique

There has been nearly a thousand year of history by using Tibetan medicated bath liquor (TMBL) made by crude drugs from Tibet in China and to treat a series of difficult and complicated cases, such as rheumatoid arthritis, rheumatic arthritis, psoriasis, postpartum convulsion, muscular atrophy, callosity, hemiplegia and hypertension. Its therapeutic effect is remarkable without any obviously toxic action and side effects. The Tibetan drug sources are rich. All of these consist of a distinguishing feature and specialty for the treatment of difficult and complicated cases in Tibetan medical therapy.

The characteristics of the above-mentioned diseases are of a longer course with disease. Not only the ordinary Chinese Traditional therapy and western medicine need a long therapeutic course for the treatment of said diseases, but also the therapeutic effect is not so ideal. However, if these diseases are treated with Tibetan medicine bath liquor through 1-3 courses of treatment, all of them obtain a better therapeutic effect. Recently a number of " Section of TMBL" have been established in many places in Tibet, Xinjiang, Inter Mongol, Gansu, Qinghai, Sichuan and Henan. However, these hospitals relate to the source of Tibetan drug and most of them are set up in the remote mountainous districts and county towns where the traffic is inconvenient, it brings about much inconvenience to the patients. On the other hand, the drug bath therapy requires the patients to be away from the wind in spring and autumn and to be treated under keeping heat, while most patients with these diseases usually have acronymic and akinesia. For these reasons, if under the direction of physician the patients can be treated and nursed at home or in the nearby hospital or clinic, it will have a positive meaning for the therapy and medical care in reducing any difficulty and expense from patients. In view of the above-mentioned facts, the dosage-form of TMBL should be improved to realize its industrialized production of medicament, in order to offer the MBL therapy to a great amount of patients and to serve them.

A paper of Jiuxi Jabu "Observation of Therapeutic Effects of Tibetan Medicated Bath Liquor in 50 Cases of Rheumatoid Disease" in «Chinese Journal of Folk Medicine» 1997,Vol.3, No.3, p.18, reported that a bath liquor containing traditional pharmaceuticals, so called "Sweet Dew Bath with five flavor" was prepared with five kinds of traditional drug, namely Junipers clavaum L., Rhododendron, Myricaria germanica (L.) Desv. Chinese Ephedra and Artemisia (wormwood). Its specific preparative method is as follows:

The above-mentioned five kinds of Chinese traditional drug are collected. After cutting them into slices and drying, the distiller's yeast is added for fermentation and dried by airing again. And also, according to the patients' condition and severity of disease, other several kinds of traditional drug are also added. The above-resulted mixture are put into a white cloth bag, soaked with water overnight and decocted. After the contents in the pot are concentrated to a half volume, the decocted liquor is poured into a vat and the drug bag is decocted once again to a volume of 2/3 of the original, and then the decocted drug liquor mixture is poured into the vat again. Decoction is repeated for another time to a volume of 1/3. The total amount of liquor from three times of decoction is combined and mixed homogeneously with highland barley. It is ready for used now.

Owing to the facts that amount of such drug liquor for medicinal use is large, a time for production is long and volume of such drug liquor is too large, even if such kind of MBL can be obtained by means of industrial production, it is still with difficulty to the patients who purchase it and take use at home. Hence the patients must be treated by hospitalization only. For this reason the dosage-form of medicated bath liquor (MBL) has been studied by some people.

In the «First Colloquium on Chinese Tibetan Drugs and Their Naturally Developing Strategy» (Papers Collection, pp.23-31), Li Fu-Yin Et al. has reported the preparative methods of TMBL. I.e., according to the formulation a crude traditional drug is selected, washed, cut into slices (5-10 mm), which is put into a multi-functional extracting jar. Water is added with an amount of 10 times as much as the total quantity of crude drugs. It is extracted under heat for one hour. Both volatile oil and a distillate containing volatile oil are filtered to obtain the 1st extract. To the drug residue water is added in the amount of 6 times the size of the total quantity of crude drug. And again it is extracted under heat for another one hour and filtered to obtain the 2nd extract. Then to the drug residue water is added in the amount of 4 times as much as the total quantity of crude drugs. It is extracted under heat for half an hour and filtered to obtain the 3rd extract. The three portions of extract are combined and concentrated under reduced pressure to form a paste extract. The latter is mixed homogeneously with volatile oil as well as a distillate containing volatile oil and this mixture is added with a suitable stabilizer and auxiliary bath liquor. Thus ointment containing 2 g of crude drug /ml is obtained. The latter can be diluted to form MBL when using. Although the advantage of this drug in the form of ointment lies in its decreasing volume, the water content of such ointment is still in more than 60%. The volume is still large to make transportation and application inconvenient. Besides, a certain amount of stabilizer is added with a result of increasing some chemical constituents and thereby the product will easily get moldy and deteriorate with poor preservation. The above-mentioned drawback will limit to the development and application of the therapy of TMBL.

Because of the deficiency in liquid form of MBL and ointment form of MBL, some people are going to do a further study on their dosage forms. A paper of Otkan Qimo et al. "An Exploration for Application and Improvement of Preparative Method of Mongolian Medicated Bath " in «Chinese Journal of National Medicine» 1997, Vol.3, No.1, p.29. Provided a preparative method of drug bath powder. First of all, a number of crude drugs, which are not suitable for extraction, such as aromatic drugs, mineral drugs and animal drugs etc., are selected and crushed. A series of botanical drugs, such as Junipers clavaum L., Artemisia (wormwood), Chinese Ephedra, Myricaria germanica (L.) Desv.Rhododendron anthopogonoides Leaves are decocted and ready for use. The method in detail: Firstly the crude drugs are decocted for 2 hrs (drug: water = 1: 5 by weight). The filtrates are corrected and again the residue is decocted for 6 hrs. Filtration and decoction are needed for 4-5 times repeatedly till the drug liquor shows colorless and tasteless. Several batches of drug liquor are combined and concentrated by heating. A paste is formed and laid up for cooling. The crushed crude drugs, which are not suitable for boiling with water, are added in. And then a powdered substance is formed after stirring , stoving and grinding. Of course, many kinds of drug bath powder can be prepared according to the different kind of disease. The methods of application with them: according to the particular disease, 150-250 g (one dose per every person) of suitable drug bath powder are mixed with water, stirred homogeneously and boiled. And then it is put into bathtub containing suitable amount of warm water and stirred homogeneously. Bath can be taken just now. The advantages of this drug bath powder are of greatly decreasing weight and volume of drug bath preparation and being convenient to carry, while the disadvantages are that the effective constituents could be destroyed during stoving and the effective volatile constituents will volatilize in a certain degree to make some losses. In addition, only boiling during the use treats the original powder of the crude drug, so the drugs cannot sufficiently produce its effectiveness and thus waste is occurred.

### Disclosure of the invention

The aim of the present invention is to overcome the drawback present in the above-mentioned liquid form of MBL and paste form of MBL, i.e. with heavy weight and large volume; inconvience of carrying; destruction of effective ingredient of drug bath powder during stoving; no producing sufficient effectiveness of the crude drugs which are added, but causing some waste. The present invention provides a method processing a MBL from natural plants to a drug powder, which is not only to be transported with convenience (due to directly soluble in water directly), but also to be applied with convenience. In short, the therapeutic effect is high without any waste.

The present invention pertains to a method of processing a medicated bath liquor (MBL) from the natural plants
selected from the group consisting of mixture of Acanthopanax Bark, Piper kadsura Ohwi, Phryma leptostachya L, Angelica, Green Tangerine Peel, Papaya, and Lycopodium clavatum L.;
mixture of Rhododendron, Artemisia Sieversiana Exhart ex Willd, Chinese Ephedra, Juniperus clavaum L. and Myricaria germanica (L.) Desv; or
mixture of Sapium Sebiferum (L.) Roxb, fresh Cinnamomum Camphora (L.) Presl, Pine leaves, and Ginger
into a drug powder, which comprises:
the pretreated natural plants are placed in the vessel for extraction, water is added with an amount of 5-15 times as much as the total quantity of crude drugs from saidnatural plants, the 1st extraction is carried out under heat (70°C to boiling temperature) for 1/2-24 hrs, the volatile oil and distillate containing volatile oil are obtained, after filtration the 1 st extract and 1 st drug residue are obtained; and then water is added into the 1st residue with an amount of 3-10 times as much as the total quantity of crude drugs from said natural plants. The 2nd extraction is carried out under heat (70°C to boiling temperature) for 1/2-5 hrs, after filtration the 2nd extract and 2nd residue are obtained, and then water is added into the 2nd residue with an amount of 3-10 times as much as the total quantity of crude drugs from the natural plants, the 3rd extraction is carried out with heat (70°C to boiling temperature) for 1/2-5 hrs after filtration the 3rd extract and 3rd residue are obtained, all three extracts are combined together; the combined extracts are concentrated under vacuum to form a concentrated medicated liquor which is further treated by freeze drying under vacuum to form a solid drug, the latter is ground to form a drug powder, or the concentrated drug liquor is dried with spraying to form a drug powder, hereafter, the above-mentioned distillate containing volatile oil is isolated through oil-water separation to get the oil, combining the obtained oil with the originally prepared volatile oil; the combined volatile oil is added into the powdered drug with homogenous mixing and the powdered drug is now completed.

### Preferred embodiments of the invention

The present invention provided a method of processing a medicated bath liquor (MBL) from a natural plant into a drug powder. The pre-treated natural plants are placed in the vessel for extraction. Water is added in an amount of 5-15 times as much as the total quantity of crude drugs from the natural plants. The 1st extraction is carried out with heat (70°C to boiling temperature) for 1/2 - 24 hrs. The volatile oil and distillate containing volatile oil are obtained. After filtration the 1st extract and 1st drug residue are obtained. And then water is added into the 1st residue with an amount of 3-10 times as much as the total quantity of crude drugs from the natural plants. The 2nd extraction is carried out with heat (70°C to boiling temperature) for 1/2-5 hrs. After filtration the 2nd extract and 2nd residue are obtained. And then water is added into the 2nd residue with an amount of 3-10 times as much as the total quantity of crude drugs from the natural plants. The 3rd extraction is carried out with heat (70°C to boiling temperature) for 1/2-5 hrs. After filtration the 3rd extract and 3rd residue are obtained. All of three extracts are combined together. The characteristics are as follows: the combining extracts are concentrated under vacuum to form a concentrated medicated liquor which is further treated by freeze drying under vacuum to form a solid drug. The latter is ground to form a drug powder, or the concentrated drug liquor is dried with spraying to form a drug powder. Hereafter, the above-mentioned distillate containing volatile oil is isolated through oil-water separation to get the volatile oil. After combining with the originally prepared volatile oil, the combination is added into the powdered drug with homogeneous mixing and the powdered preparation is now completed. Because the quantity of volatile oil, which is added, is less, the final product is still a powdered drug.

The present invention provides a method of processing the powdered Drug with a medicated bath liquor (MBL) from the natural plants. Furthermore, water is added again into the 3rd residue with an amount of 3-10 times as much as the total quantity of crude drugs. The 4th extraction is carried out with heat (70°C to boiling temperature) for 1/2-5 hrs. After filtration the 4th extract is obtained. Four parts of extract are combined and concentrated under vacuum to form concentrated medicated liquor, which is further treated by freeze- drying under vacuum to form a solid drug. The latter is ground to form a drug powder, or the concentrated drug liquor is dried with spraying to form a drug powder. Hereafter, the above-mentioned distillate containing volatile oil is isolated through oil-water separation to get a volatile oil. After combining with the originally prepared volatile oil, the combination is added into the powdered drug with homogenous mixing and the powdered preparation is now completed.

For the first time, water is added into the pretreated raw materials (crude drugs of natural plants) with an amount of 5-10 times as much as them. First extraction under heat at 70°C to boiling temperature for 1.5-6 hrs. The result is the best;

The said pressure for concentrating the combined extracts is 0.01- 0.09 Mpa, and 0.01-0.06 Mpa is preferred. The temperature for concentrating the combining extracts under vacuum is 60°C-90°C;

The extract is concentrated to a volume of 1/10-1/2 as much as that of combined extracts to form a concentrated medicated liquor;

When the concentrated medicated liquor is dried to form powdered drug by spray, water steam is as spray gas, the pressure of spray gas is 0.1-1.0 Mpa and the temperature for drying is 60°C-500°C;

Freeze-drying for concentrated drug liquor is carried out under the pressure of 10-800 Pa, and the temperature for freeze-drying is -5°C to -50 °C;

Water content in the prepared powdered drug is 5-10% of the total weight of powdered drug.

During the spray-drying for the concentrated drug liquor to form dried powdered drug, the concentrated drug liquor is sprayed into a hot air chamber with a centrifugal spray and the hot air of 60°C- 400°C is as a drying medium. An instantaneous heat exchange is going on and drying thus forms the powdered drug. The temperature for spray-drying is 200°C-400°C.

The said pretreatment of raw materials from the natural plants is as follows: the crude drugs from natural plants are selected as raw materials, which are qualified in properties. They are washed with water, dried in air and cut into slices of a size of 5-20 mm (5-10 mm slices is preferred). Some crude drugs must be soaked again before hot extraction, so it is advantageous to dissolve the drugs out when extracting under heating. The time of soaking depends on the property of crude drugs; it is a knowledge known to the skilled person in this art. For example, it is sufficient for some raw materials to soak for 30-40 min and others for 12-24 hrs. Sometimes water after soaking can be utilized as a part of water firstly added. After sometime the sliced crude drugs are washed and dried in air, it is necessary to make fermentation with yeast and dry in air again. In general MBL needs fermentation with yeast before use, because the therapeutic effects after fermenting will be better for treating rheumatic disease, postpartum convulsion, apoplectic sequel and dermatitis. The specific preparative method is as follows: the sliced crude drug is added into a steamer to be boiled for 30-60 min. The contents in a steamer are taken out and cooled to 30°C-40°C in air. After the yeast is mixed with it, the mixture stands for 48-72 hrs at 30°C-40°C (keeping 30°C-40°C) until a alcohol odor appears. For example, Six crude drugs, such as Rhododendron anthopogonoides, Mercurial Germanic (L.) Desv.Juniperus clavaum L., Chinese Ephedra, a kind of Artemisia ,Yanthoceras sorbifolia Bunge. etc., for treating rheumatoid arthritis are fermented with yeast for 3 days. (See " Clinical Observation on 223 Cases of Rheumatoid arthritis by Treatment with Tibetan Drug Bath", «Chinese Journal of National Medicine» 1997, Vol.3, No.2, p.15. The above-mentioned pretreatment method is known to the skilled person in the art. Water for the prefreatment may be tap water and well water or distilled water as well as ion-exchanged water, the latter two are purer water treated by artificial processes.

Owing to that it is difficult to extract active ingredient from some botanical crude materials, it is necessary to add water into the 3rd drug residue. The amount of water is 3-10 times as much as the total amount of a crude material from natural plants. The 4th extraction is carried out under heat at 70°C to boiling temperature for 1/2-5 hrs. After filtration the 4th extract and the 4th drug residue are obtained. Sometimes it is necessary to do for 5th, 6th and 7th extraction, the times of extruction depend on the specific circumstance and finally the drug residues are discarded. After combining 1st, 2nd, 3rd and 4th extract or 1st, 2nd, 3rd, 4th, 5th......extract and concentrated under reduced pressure, lyophilization and grinding or spray drying are carried out to form a powdered drug. Finally a volatile oil is added into the powdered drug. All of a crude material from a natural plant contain the volatile oil with different content and those of low content are not worth extracting. If so, the procedures of collecting the volatile oil during extraction and then adding the volatile oil into the powdered drug can be omitted.

Owing to the active ingredient of some botanical crud material can be easily extracted with water, only 1st extraction or 1st and 2nd extraction are carried out only and the times of extraction are depended on the property of botanical crude drugs.

When water is firstly added into the pretreated crude materials from natural plants, water is added with an amount of 5-15 times as much as the total quantity of a crude material from a natural plant, preferably 5-10 times. The first extraction under heat at 70°C to boiling temperature for 1.5-6 hrs is preferred. When concentration under reduced pressure, pressure is 0.01-0.09 Mpa, preferably 0.01-0.06 Mpa. The temperature for concentration under reduced pressure is 60°C-90°C. The volume of the concentrated liquor under reduced pressure is 1/10 - 1/2 time as much as that of the extract. The pressure for freeze-drying of concentrated extract liquor under vacuum is 10-800 pa. The temperature for lyophilization is -5°C to -50°C. Water content of the powdered drug is 5-10%. During spray-drying for the concentrated extract liquor, water steam is as spray gas for spraying, the pressure of spray is 0.1-1.0 Mpa. The temperature for drying is 60°C-500 °C, preferably 200°C-400°C. Besides, when dried with spraying, the concentrated extract liquor is sprayed into a hot air chamber by a centrifugal spray and the hot air of 60°C-400°C is as a drying medium, and instantaneous heat exchange is going on, and the powdered drug is thus formed. To the drug powder the volatile oil is added.

The container for extraction is decocting pot, multi-function extracting pot. Both of them have a water condenser. During the 1st extraction with water the volatile oil and water condense as a condensate. The volatile oil is separated with an oil -water separator and loaded in the bottle for use. Finally, the volatile oil is added into the powdered drug which has been ground. After freeze-drying a powdered product is formed.

According to the method of the present invention and the cases of different diseases, the powdered drug which is made from the composition of different botanic crude material is used to treat different diseases.

The powdered drugs prepared by the method of the present invention, every gram of powdered drug contains the active ingredient which is equal to 10 g of crude material. The amount of active has greatly exceeded the content of active ingredient from the crude material in the pharmaceutical preparation made by the known method.

The advantages of method provided by the present invention are as follows :
1. The methods of the present invention is more stable than that of the know method and the obtained drug powder is higher and maybe controlled with respect of quality. These factors are favourable to guarantee the therapeutical effects. A detection standard can be set up for quality examination (the above-mentioned three methods have no standard astoquality). The content of drug and active ingredient can be determined in order to guarantee the quality of product, the powdered drug of TMBL.
2. Owing to the facts that the method of the present invention uses the modem technique of freeze under vacuum and spray-drying, not only the active ingredient from the natural plants can be effectively reserved, but also the ratio of crude material is greatly increased to give a better therapeutic effect and the prolonged storage period of drugs. Therefore it is convenient for domestic use.
3. It needs not adding any chemically synthetic stablizer into the product of powdered drug prepared by the method of the present invention. Therefore, it is safe and convenient because warm water is added only when using. Improvement of dosage form of the present invention impels to realize industralization in producing TMBL.

**The following examples is used only to further illustrate the present invention but not intend to produce any limitation to the present invention**

### Example 1 : Preparation of the medicated bath powder for the treatment of arthralgia-symdrome

A crude material from a natural plant in this prescription includes : Acanthopanax Bark 20 g, Piper kadsura (choisy) Ohwi 20 g, Tou Feng Cao (a kind of herb) 20 g, Angelica 10 g, Green Tangerine Peel 10 g, Papaya 10 g, Lycopodium clavatum L. 10 g.

Firstly the above-mentioned botanical crude materials are pretreated with the method as follows:

The qualified crude materials are selected , washed with water, dried in air, cut into slices in a size of 6 mm, soaked with water for 12 hrs. The above-mentioned and pretreated crude materials are put into a multi-functioned extracting pot. Water is added for first time. The water added is well water for drinking in an amount of 9 times , 5 times, 3 times or 10 times as much as the total weight of crude materials.

The 1st extraction with heat is carried out at the temperature of 70°C to boiling temperature for 3 hrs, 1/2 hr or 5hrs, and then the volatle oil and the distillate containing volatile oil are obtained. The distillate containing voaltile oil is treated by oil-water separation and the obtained volatile oil is mixed with the above-mentioned volatile oil and loaded in bottles for use. After filtration the 1st extract and 1st drug residue are obtained. Water is added into the 1st drug residue with an amount of 3 times , 10 times or 5 times as much as the total weight of crude materials.

The 2nd extraction with heat is carried out at the temperature of 70°C to boiling temperature for 1.5 hr, 1/2 hr or 5hrs. After filtration the 2nd extract and 2nd drug residue are obtained. Water is added into 2nd residue with an amount of 3 times, 10 times or 5 times as much as the total weight of crude drugs.

The 3rd extraction with heat is carried out at the temperature of 70°C to boiling temperature for 1 hr, 1/2 hr or 5 hrs. After filtration the 3rd extract and 3rd drug residue are are obtained. Of course, according to the concrete conditions water may be added into 3rd residue with an amount of 3 times, 10 times or 5 times as much as the total weight of crude drugs.

The 4th extraction with water is carried out at the temperature of 70°C to boiling temperature. Finally the residues are discarded and the 1st, 2nd and 3rd extracts or 1st, 2nd, 3rd and 4th extracts are combined and concentrated under the reduced pressure at 0.05" or 0.01MPa, or 0.06 or 0.09 MPa. The temperature for concentration under reduced pressure is 70 ± 10°C or 90°C. The drug liquor is concentrated to 1/5, 1/10 or 1/2 of the original volumn of extract. Freeze-drying under vacuum is carried out to form a solid. The pressure for freeze-drying under vacuum is 30 Pa, 10Pa or 800Pa, while then temperature for freeze-drying under vacuum is -10°C, -5 °C or -50°C. The water content of thus formed solid drug is 6.1%, 5% or 10% of the total weight of the solid drug. And the solid drug formed by freeze-drying under vacuum is ground to form a powder. The above-mentioned volatile oil is incorporated homogeneously into a drug powder, The medicated bath powder for the treatment of arthralgia syndrome is thus prepared.

### Example 2 : Using the preparative method provided by the present inventon to prepare drug bath powder for treatng rheumatism, rheumaloid arthritis and skin disease :

This example illustrates a combining prescription of medicated bath used by Tibetan physicians to treat rheumatism, rheumatoid disease and skin disease. In this prescription the raw materials from the natural plants include : Rhododendron 100 g, Artemisia Sieversiana Exhart ex Willd 100 g, Chinese Ephedra 100 g, Juniperus clavaum L. 100 g and Myricaria germanica (L.) Desv. 100 g.

Firstly the above-mentioned botanical raw materials are pretreated with the method as follows : The qualified raw materials are selected , washed with water, dried in air, cut into slices in a size of 6 mm or so. Then it is put into a steamer to be boiled for 40 min. The raw materials are taken out from steamer and cooled to 35°C in air, and then mixed and stirred with a suitable amount of yeast. The obtained mixture is maintained at 35°C for 60hrs till the alcohol smell appears.

The remaining procedures are basically identical with those in Example 1. The differences lie in the prescription of raw materials in MBL, the diseases to be treated and the method of powder formation.

By means of the method of heat extraction in Example 1, the above-mentioned pretreated raw materials are extracted with heat for 3 times and three portions of extracts are combined, concentrated under reduced pressure in 0.03 Mpa at 65°C±5°C. After the drug liquor is concentrated to a volumn of 1/3 (circa) as much as that of combined extracts, spray-drying is carried out to form the solid drug. Its condition is as follows: water steam is applied as nebulization gas under the pressure of 0.4 Mpa, 0.1 Mpa or 1.0 Mpa. The temperature for spray-drying is 400°C, 60°C, 500 °C or 200°C. The dried solid drug is then ground into drug powder. An another method is as follows: when the concentrated drug liquor is dried with spray to form a powdered drug, the liquor is sprayed into a hot air chamber with a centrifugal spray and the hot air of 60°C-400°C is as a drying medium, and an instantaneous heat exchange is occured, the concentrated liquor is dried to form powdered drug , the volatile oil is added into it. The medicated bath powder for the treatment of rheumatism, rheumatoid arthritis and skin diseases is thus formed.

### Example 3 : Preparation of medicated bath powder for treating skin diseases

The preparative and operative procedures are basically indentical with that of Example 1. The differences lie in drug conponents and diseases to be treated. This example illustrates a combining prescription of medicated bath used by Tibetan physicians to treat skin diseases. In this prescription the crude drug from natural plants included : Sapium Sebiferum(L.) Roxb 60 g, fresh Cinnamomum Camphora (L.) Presl, 60 g, Pine leaves 60 g, Ginger 60 g.

Firstly the above-mentioned botanical crude drugs are pretreated with the method as follows : the above-mentioned crude drugs with good quality is washed with water, dried in air, cut into slices in 9 mm or so and is ready for use.

The pretreated crude drugs from natural plants are placed in a multi-functional pot and water is added in an amount of 10 times as much as the total weight of crude drugs. The 1st extraction under heat is carried at boiling temperature for 6 hrs and volatile oil as well as distillate are obtained. The distillate containing volatile oil is treated by oil-water separation. The volatile oil from distillate is obtained, and mixed with the above-mentioned volatile oil and loaded in the bottle. After filtration the 1st extract and 1st residue are obtained. Water is added into the 1st residue with an amount of 7 times as much as the total quantity of crude drugs. The 2nd extraction under heat is carried out at boiling temperature for 1.5 hr. After filtration the the 2nd extract and 2nd residue are obtained. Water is added into the 2nd residue with an amount of 4 times as much as the total quantity of crude drugs. The 3rd extraction under heat is carried out at boiling temperature for 0.7 hr. After filtration the 3rd extract and 3rd residue are obtained and the 3rd residue is discarded. The 1st, 2nd, and 3rd extract are combined and concentrated under reduced pressure. The pressure for concentration under reduced pressure is 0.07 Mpa, while the temperature for concentration under reduced pressure is 70°C ± 5°C. After the drug liquor is concentrated to a volumn of 0.15 time as much as volumn of the combined extracts, freeze-drying under vacuum is carried out to form the solid drug. The pressure for freeze-drying under vacuum is 50Pa and the temperature for freeze-drying is -25°C. Water content of the formed solid drug powder is 7%. And again the solid drug formed by freeze-drying under vacuum is ground to form a drug powder. Finally the volatile oil in the above-mentioned bottle is added into the drug powder and the product is mixed homogeneously. The drug bath powder of this example which is used for treating skin diseases is thus provided

## Claims

1. A method of processing a medicated bath liquor (MBL) from the natural plants
selected from the group consisting of mixture of Acanthopanax Bark, Piper kadsura Ohwi, Phryma leptostachya L, Angelica, Green Tangerine Peel, Papaya, and Lycopodium clavatum L.;
mixture of Rhododendron, Artemisia Sieversiana Exhart ex Willd, Chinese Ephedra, Juniperus clavaum L. and Myricaria germanica (L.) Desv; or mixture of Sapium Sebiferum (L.) Roxb, fresh Cinnamomum Camphora (L.) Presl, Pine leaves, and Ginger
into a drug powder, which comprises:
the pretreated natural plants are placed in the vessel for extraction, water is added with an amount of 5-15 times as much as the total quantity of crude drugs from said natural plants, the 1st extraction is carried out under heat (70°C to boiling temperature) for 1/2 - 24 hrs, the volatile oil and distillate containing volatile oil are obtained, after filtration the 1st extract and 1st drug residue are obtained; and then water is added into the 1st residue with an amount of 3-10 times as much as the total quantity of crude drugs from said natural plants. The 2nd extraction is carried out under heat (70°C to boiling temperature) for 1/2-5 hrs, after filtration the 2nd extract and 2nd residue are obtained; and then water is added into the 2nd residue with an amount of 3-10 times as much as the total quantity of crude drugs from the natural plants, the 3rd extraction is carried out with heat (70°C to boiling temperature) for 1/2-5 hrs after filtration the 3rd extract and 3rd residue are obtained, all three extracts are combined together; the combined extracts are concentrated under vacuum to form a concentrated medicated liquor which is further treated by freeze drying under vacuum to form a solid drug, the latter is ground to form a drug powder or the concentrated drug liquor is dried with spraying to form a drug powder, hereafter, the above-mentioned distillate containing volatile oil is isolated through oil-water separation to get the oil, combining the obtained oil with the originally prepared volatile oil; the combined volatile oil is added into the powdered drug with homogenous mixing and the powdered drug is now completed.

2. A method according to Claim 1, which is **characterised in that**: water is added again into the 3^{rd} residue with an amount of 3-10 times as much as the total quantity of crude drugs, the 4^{th} extraction is carried out with heat (70°C to boiling temperature) for 1/2-5 hrs, after filtration the 4^{th} extract is obtained, four parts (1^{st}, 2^{nd}, 3^{rd} and 4^{th}) of extract are combined and concentrated under vacuum to form a concentrated medicated liquor which is further treated by freeze-drying under vacuum to form a solid drug, the latter is ground to form a drug powder, or the concentrated drug liquor is dried by spraying to form a drug powder, hereafter, the above-mentioned distillate containing volatile oil is isolated through oil-water separation to get the oil, after combining the obtained oil with the originally prepared volatile oil which is ready for use, the combined volatile oil are added into the powdered drug with homogenous mixing and the powdered drug is now completed.

3. A method according to Claim 1, which is **characterised in that**: water is firstly added into the crude drugs of natural plants with an amount 5-15 times as much as the total quantity of crude drugs from the natural plants, the 1^{st} extraction is carried out with heat (70°C to boiling temperature) for 1.5-6 hrs.

4. A method according to Claim 1 or 2 which is **characterised In that**: the pressure for concentrating the combining extracts under reduced pressure is 0.01-0.09 MPa and the temperature is 60°C-90°C.

5. A method according to Claim 4 which is **characterised in that**: the pressure for concentrating the combining extract under reduced pressure is 0.01-0.06 MPa.

6. A method according to Claim 4 which is **characterised in that**: the volume of concentrated drug liquor is 1/10 to 1/2 volume of the combined extract, and a concentrated drug liquor is formed.

7. A method according to Claim 1 which is **characterised in that**: when concentrated medicated liquor is dried to form powdered drug by spraying, water steam as spray gas, the pressure is 0.1-1.0 MPa and the temperature for drying is 60°C-500°C;

8. A method according to Claim 1 which is **characterised in that**: the pressure for freeze-drying concentrated medicated liquor under vacuum is 10-800 Pa and the temperature is -5°C to -50°C,

9. A method according to Claim 1 which is **characterised in that**: the water content in such formed powdered drug is 5-10 % of the total weight of powdered drug.

10. A method according to Claim 1 which is **characterised in that**: when the concentrated drug liquor is dried with spray to form a powdered drug, the liquor is sprayed to a hot air chamber with a centrifugal sprayer and the hot air of 60°C-400°C as a drying medium, An instantaneous heat exchange is occuring and the powdered drug is thus formed by drying,

11. A method according to Claim 7, which is **characterised in that**: the temperature for drying concentrated drug liquor with spray to form a powdered drug is 200°C-400°C.

## Patentansprüche

1. Verfahren zur Verarbeitung einer medizinischen Badeflüssigkeit (MBL) aus Naturpflanzen,
ausgewählt aus der Gruppe, bestehend aus einer Mischung aus Acanthopanax Rinde, Piper kadsura Ohwi, Phryma leptostachya L, Angelika, grüner Mandarinenschale, Papaya und Lycopodium clavatum L;
einer Mischung von Rhododendron, Artemisia Sievrsiana Exhart ex Willd, chinesischen Meerträubchen (Ephedra), Juniperus clavaum L und Myricaria germanica (L.) Desv; oder einer Mischung aus Sapium Sebiferum (L.) Roxb, frischen Cinnamomum Camphora (L.) Presl, Pinienlaub und Ingwer,
in ein Arzneimittelpulver, welches umfasst:
die vorbehandelten Naturpflanzen werden zur Extraktion in ein Gefäß eingebracht, Wasser wird in 5 - 15 facher Menge der Gesamtmenge der rohen Arzneimittel von den Naturpflanzen zugegeben, die erste Extraktion wird unter Wärme (70°C bis Siedetemperatur) für 1/2 - 24 h durchgeführt, das flüchtige Öl und das Destillat, das flüchtiges Öl enthält, werden erhalten, nach Filtration werden der erste Extrakt und der erste Arzneimittelrückstand erhalten, und dann wird Wasser zu dem ersten Rückstand mit 3-10 facher Menge der Gesamtmenge der rohen Arzneimittel von den Naturpflanzen zugegeben. Die zweite Extraktion wird unter Wärme (70°C bis Siedetemperatur) für 1/2 -. 5 h durchgeführt, nach Filtration werden der zweite Extrakt und ein zweiter Rückstand erhalten, und dann wird Wasser zu dem zweiten Rückstand in 3 - 10-facher Mengeder Gesamtmenge der rohen Arzneimittel von den Naturpflanzen zugegeben, die dritte Extraktion wird unter Wärme (70°C bis Siedetemperatur) für 1/2 - 5 h durchgeführt, nach Filtration werden der dritte Extrakt und der dritte Rückstand erhalten, alle drei Extrakte werden miteinander kombiniert, die kombinierten Extrakte werden unter Vakuum aufkonzentriert, um eine konzentrierte medizinische Flüssigkeit zu bilden, die weiterhin unter Vakuum gefriergetrocknet wird, um ein festes Arzneimittel zu bilden, das Letztere wird gemahlen, um ein Arzneimittelpulver zu bilden, oder die konzentrierte Arzneimittelflüssigkeit wird sprühgetrocknet, um ein Arzneimittelpulver zu bilden, hiernach wird das zuvor genannte Destillat, das flüchtiges Öl enthält, durch Öl-Wasser-Separation isoliert, um das Öl zu gewinnen, Kombinieren des erhalten Öls mit dem ursprünglich hergestellten flüchtigen Öl; das kombinierte Öl wird durch homogenes Mischen zum pulverisierten Arzneimittel gegeben und das pulverisierte Arzneimittelpulver ist nun fertig.

2. Verfahren nach Anspruch 1, welches **dadurch gekennzeichnet ist**: Wasser wird wiederum zu dem dritten Rückstand in 3 - 10 facher Menge der Gesamtmenge der rohen Arzneimittel gegeben, die vierte Extraktion wird mit Wärme (70°C bis Siedetempertur) für 1/2 - 5 h durchgeführt, nach Filtration wird der vierte Extrakt erhalten, vier Teile (Erster, Zweiter, Dritter und Vierter) des Extrakts werden kombiniert und unter Vakuum aufkonzentriert, um eine konzentrierte medizinische Flüssigkeit zu bilden, die weiterhin unter Vakuum gefriergetrocknet wird, um ein festes Arzneimittel zu bilden, das Letztere wird gemahlen, um ein Arzneimittelpulver zu bilden, oder die konzentrierte Arzneimittelflüssigkeit wird sprühgetrocknet, um ein Arzneimittelpulver zu bilden, hiernach wird das zuvor genannte Destillat, das flüchtiges Öl enthält, durch Öl-Wasser-Separation isoliert, um das Öl zu gewinnen, nach Kombinieren des erhaltenen Öls mit dem ursprünglich hergestellten flüchtigen Öl , das gebrauchsfertig ist, wird das kombinierte flüchtige Öl zu dem pulverisierten Arzneimittel unter homogenem Mischen gegeben und das pulverisierte Arzneimittel ist nun fertig.

3. Verfahren nach Anspruch 1, welches **dadurch gekennzeichnet ist**: Wasser wird zunächst zu den rohen Arzneimitteln der Naturpflanzen in 5 - 15-facher Menge der Gesamtmenge der rohen Arzneimittel von den Naturpflanzen zugegeben, die erste Extraktion wird unter Wärme (70°C bis Siedetemperatur) für 1,5 - 6 h durchgeführt.

4. Verfahren nach Anspruch 1 oder 2, welches **dadurch gekennzeichnet ist**: der Druck zum Aufkonzentrieren der kombinierten Extrakte unter reduziertem Druck ist 0,01 - 0,09 MPa und die Temperatur ist 60°C - 90°C.

5. Verfahren nach Anspruch 4, welches **dadurch gekennzeichnet ist**: der Druck zum Aufkonzentrieren der kombinierten Extrakte unter reduziertem Druck ist 0.01 - 0,06 MPa.

6. Verfahren nach Anspruch 4, welches **dadurch gekennzeichnet ist**: das Volumen der aufkonzentrierten Arzneimittelflüssigkeit ist 1/10 bis 1/2 Volumen des kombinierten Extrakts und eine aufkonzentrierte Arzneimittelflüssigkeit wird gebildet.

7. Verfahren nach Anspruch 1, welches **dadurch gekennzeichnet ist**: wenn die aufkonzentrierte medizinische Flüssigkeit durch Sprühen getrocknet wird, um ein pulverisiertes Arzneimittel zu bilden, Wasserdampf als Sprühgas, der Druck ist 0,1 - 1,0 MPa und die Temperatur zum Trocknen ist 60°C - 500°C.

8. Verfahren nach Anspruch 1, welches **dadurch gekennzeichnet ist**: der Druck zum Gefriertrocknen der aufkonzentrierten medizinischen Flüssigkeit unter Vakuum ist 10 - 800 Pa und die Temperatur ist -5°C bis -50°C.

9. Verfahren nach Anspruch 1, welches **dadurch gekennzeichnet ist**: der Wassergehalt bei einem solchen pulverisiertes Arzneimittel ist 5 - 10 % des Gesamtgewicht des pulverisiertes Arzneimittelpulvers.

10. Verfahren nach Anspruch 1, welches **dadurch gekennzeichnet ist**: wenn die aufkonzentrierte Arzneimittelflüssigkeit durch Sprühen getrocknet wird, um ein pulverisiertes Arzneimittel zu bilden, wird die Flüssigkeit mit einem Zentrifugalsprüher in eine heiße Kammer gesprüht und die heiße Luft von 60°C - 400°C als ein Trocknungsmedium, ein plötzlicher Wärmeaustausch tritt auf und das pulverisiertes Arzneimittel wird so durch Trocknen gebildet.

11. Verfahren nach Anspruch 7, welches **dadurch gekennzeichnet ist**: die Temperatur zum Sprühtrocknen der aufkonzentrierten Arzneimittelflüssigkeit ist 200°C - 400°C, um ein pulverisiertes Arzneimittel zu bilden.

## Revendications

1. Procédé de traitement d'une liqueur de bain médicinale (MBL) provenant de plantes naturelles
choisies dans le groupe constitué par un mélange de Acanthopanax Bark, de Piper Kadsura Ohwi, de Phryma Leptostachya L., d'Angelica, de Green Tangerine Peel, de papaye et de Lycopodium clavatum L. ;
un mélange de Rhododendron, d'Artemisia Sieversiana Exhart ex Willd, d'éphédra de Chine, de Juniperus clavaum L. et de Myricaria germanica (L.) Desv ; ou
un mélange de Sapium Sebiferum (L.) Roxb, de Cinnamomum Camphora (L) Presl frais, de feuilles de pin et de gingembre
en un médicament en poudre, qui comprend les étapes suivantes :
les plantes naturelles prétraitées sont placées dans un récipient pour une extraction, de l'eau est ajoutée dans une quantité de 5 à 15 fois la quantité totale de médicaments bruts provenant desdites plantes naturelles, la première extraction est réalisée sous chauffage (70°C à la température d'ébullition) pendant 1/2 à 24 h, l'huile volatile et un distillat contenant l'huile volatile sont obtenus, après filtration, le premier extrait et un premier résidu de médicament sont obtenus ; et ensuite de l'eau est ajoutée au premier résidu dans une quantité de 3 à 10 fois la quantité de médicaments bruts provenant desdites plantes naturelles. La deuxième extraction est réalisée sous chauffage (70°C à la température d'ébullition) pendant 1/2 à 5 h, après filtration, le deuxième extrait et un deuxième résidu sont obtenus ; et ensuite de l'eau est ajoutée au deuxième résidu dans une quantité de 3 à 10 fois la quantité de médicaments bruts provenant des plantes naturelles, la troisième extraction est réalisée sous chauffage (70°C à la température d'ébullition) pendant 1/2 à 5 h, après filtration, le troisième extrait et un troisième résidu sont obtenus, et les trois extraits sont combinés ensemble ; les extraits combinés sont concentrés sous vide pour former une liqueur médicinale concentrée qui est en outre traitée par lyophilisation sous vide pour former un médicament solide, ce dernier est broyé pour former une poudre de médicament, ou la liqueur de médicament concentrée est séchée par atomisation pour former une poudre de médicament ; par la suite, le distillat mentionné ci-dessus contenant l'huile volatile est isolé par une séparation huile-eau pour obtenir l'huile, l'huile obtenue est combinée avec l'huile volatile préparée à l'origine ; l'huile volatile combinée est ajoutée au médicament en poudre avec un mélange homogène et le médicament en poudre est maintenant terminé.

2. Procédé selon la revendication 1, **caractérisé en ce que** : de l'eau est de nouveau ajoutée au troisième résidu dans une quantité de 3 à 10 fois la quantité de médicaments bruts, la quatrième extraction est réalisée sous chauffage (70°C à la température d'ébullition) pendant 1/2 à 5 h, après filtration, le quatrième extrait est obtenu, les quatre parties (première, deuxième, troisième et quatrième) de l'extrait sont combinées et concentrées sous vide pour former une liqueur médicinale concentrée qui est en outre traitée par lyophilisation sous vide pour former un médicament solide, ce dernier est broyé pour former une poudre de médicament, ou la liqueur de médicament concentrée est séchée par atomisation pour former une poudre de médicament ; par la suite, le distillat mentionné ci-dessus contenant l'huile volatile est isolé par une séparation huile-eau pour obtenir l'huile, après combinaison, l'huile obtenue avec l'huile volatile préparée à l'origine qui est prête à l'emploi, l'huile volatile combinée est ajoutée au médicament en poudre avec un mélange homogène et le médicament en poudre est maintenant terminé.

3. Procédé selon la revendication 1, **caractérisé en ce que** : de l'eau est d'abord ajoutée aux médicaments bruts des plantes naturelles dans une quantité de 5 à 10 fois la quantité de médicaments bruts provenant des plantes naturelles, la première extraction est réalisée sous chauffage (70°C à la température d'ébullition) pendant 1,2 à 6 h.

4. Procédé selon la revendication 1 ou 2, **caractérisé en ce que** : la pression pour la concentration des extraits combinés sous pression réduite est de 0,01 à 0,09 MPa et la température est de 60°C à 90°C.

5. Procédé selon la revendication 4, **caractérisé en ce que** : la pression pour la concentration de l'extrait combiné sous pression réduite est de 0,01 à 0,06 MPa

6. Procédé selon la revendication 4, **caractérisé en ce que** : le volume de la liqueur de médicament concentrée est de 1/10 à 1/2 du volume de l'extrait combiné et la liqueur de médicament concentrée est formée.

7. Procédé selon la revendication 1, **caractérisé en ce que** : lorsque la liqueur médicinale concentrée est séchée pour former le médicament en poudre par atomisation, la pression de la vapeur d'eau, en tant que gaz d'atomisation, est de 0,1 à 1,0 MPa et la température du séchage est de 60°C à 500°C.

8. Procédé selon la revendication 1, **caractérisé en ce que** : la pression pour la lyophilisation de la liqueur médicinale concentrée sous vide est de 10 à 800 Pa et la température est de -5°C à -50°C.

9. Procédé selon la revendication 1, **caractérisé en ce que** : la teneur en eau du médicament en poudre ainsi formé est de 5 à 10 % du poids total du médicament en poudre.

10. Procédé selon la revendication 1, **caractérisé en ce que** : lorsque la liqueur de médicament concentrée est séchée par atomisation pour former le médicament en poudre, la liqueur est atomisée dans une chambre à air chaud avec un atomisateur centrifuge et de l'air chaud à une température de 60°C à 400°C en tant que milieu de séchage, un échange de chaleur instantané a lieu et le médicament en poudre est ainsi formé par séchage.

11. Procédé selon la revendication 7, **caractérisé en ce que** : la température pour le séchage de la liqueur de médicament concentrée par atomisation pour former un médicament en poudre est de 200°C à 400°C.
